(19) Europäisches Patentamt
European Patent Office
Office européen des brevets

(11) **EP 2 912 469 B1**

(12) **EUROPÄISCHE PATENTSCHRIFT**

(45) Veröffentlichungstag und Bekanntmachung des Hinweises auf die Patenterteilung:
**27.09.2017 Patentblatt 2017/39**

(21) Anmeldenummer: **13780171.8**

(22) Anmeldetag: **24.10.2013**

(51) Int Cl.:
***G01N 33/579*** (2006.01)

(86) Internationale Anmeldenummer:
**PCT/EP2013/072328**

(87) Internationale Veröffentlichungsnummer:
**WO 2014/064221 (01.05.2014 Gazette 2014/18)**

(54) **VERFAHREN ZUR DETEKTION VON ENDOTOXINEN UND/ODER 1,3-BETA-D-GLUCANEN IN EINER PROBE**

METHOD FOR DETECTING ENDOTOXINS AND/OR 1,3-BETA-D-GLUCANS IN A SAMPLE

PROCÉDÉ DE DÉTECTION D'ENDOTOXINES ET/OU DE 1,3-BETA-D-GLUCANES DANS UN ÉCHANTILLON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.10.2012 DE 102012110288**

(43) Veröffentlichungstag der Anmeldung:
**02.09.2015 Patentblatt 2015/36**

(73) Patentinhaber: **Leibniz-Institut für Neue Materialien gemeinnützige GmbH
66123 Saarbrücken (DE)**

(72) Erfinder:
• **KUCKI, Melanie
CH-9304 Bernhardzell-Waldkirch (CH)**
• **KRAEGELOH, Annette
66440 Blieskastel-Brenschelbach (DE)**

(74) Vertreter: **Patentanwälte Gierlich & Pischitzis Partnerschaft mbB
Gerbermühlstraße 11
60594 Frankfurt am Main (DE)**

(56) Entgegenhaltungen:
EP-A1- 2 233 927     WO-A1-2011/104871

• MARINA A DOBROVOLSKAIA ET AL: "Ambiguities in applying traditional Limulus Amebocyte Lysate tests to quantify endotoxin in nanoparticle formulations", NANOMEDICINE, Bd. 5, Nr. 4, 1. Juni 2010 (2010-06-01), Seiten 555-562, XP055095850, ISSN: 1743-5889, DOI: 10.2217/nnm.10.29
• Melanie Kucki: "NanoKon-INM Detection and semi-Quantification of Endotoxin Contaminations in Nanoparticle Suspensions Subtitle", , 3. August 2012 (2012-08-03), XP055095846, Gefunden im Internet: URL:http://nanopartikel.info/files/content /dana/Dokumente/Projekte/SOPs/Nanokon SOP 2 2 2_geschützt.pdf [gefunden am 2014-01-10] in der Anmeldung erwähnt
• KATSUMI YABUSAKI ET AL: "Simplified preparation of crude and functional coagulogen by thermal inactivation of serine proteases inamebocyte lysate and its application for rapid endotoxin determination", JOURNAL OF BIOSCIENCE AND BIOENGINEERING, ELSEVIER, AMSTERDAM, NL, Bd. 113, Nr. 3, 2. November 2011 (2011-11-02), Seiten 406-411, XP028463097, ISSN: 1389-1723, DOI: 10.1016/J.JBIOSC.2011.11.002 [gefunden am 2011-11-09]

EP 2 912 469 B1

- **DARKOW R ET AL: "FUNCTIONALIZED NANOPARTICLES FOR ENDOTOXIN BINDING IN AQUEOUS SOLUTIONS", BIOMATERIALS, ELSEVIER SCIENCE PUBLISHERS BV., BARKING, GB, Bd. 20, Nr. 14, 1. Januar 1999 (1999-01-01), Seiten 1277-1283, XP001016284, ISSN: 0142-9612, DOI: 10.1016/S0142-9612(99)00022-8**

**Beschreibung**

Gebiet der Erfindung

Die Erfindung betrifft ein Verfahren zum Nachweis von Endotoxinen und/oder 1,3-β-D-Glucanen, ein Kit zur Durchführung des Verfahrens und die Verwendung von Partikeln in einem solchem Verfahren.

Stand der Technik

Mikrobiologische Verunreinigungen in Produkten können beim Menschen schwere Krankheiten auslösen. Dabei spielen insbesondere gramnegative Bakterien eine wichtige Rolle. Die Prüfung auf solche Bakterien, bzw. ihrer Zerfallsprodukte, ist für viele Produkte, beispielsweise für Medizinprodukte, vorgeschrieben.

Eine wichtige Rolle für die Toxizität dieser Bakterien spielen die Endotoxine. Dies sind Bestandteile der Zellmembran gramnegativer Bakterien. Sie werden insbesondere beim Zerfall der Bakterien freigesetzt.

Es wurden unterschiedliche Verfahren entwickelt, diese Endotoxine nachzuweisen. Viele der Methoden beruhen auf der Auslösung der Gerinnungskaskade in dem Blut von Pfeilschwanzkrebsen (*Limulidae*), insbesondere *Limulus polyphemus* und *Tachypleus tridentatus* durch die Endotoxine. Dazu werden Lysate von Blutzellen (Amöbozyten) verwendet. Der Test wird entsprechend als *Limulus*-Amöbocyten-Lysat-Test (LAL, bzw. TAL bei Verwendung von *Tachypleus tridentatus*) bezeichnet.

Die Auslesung der Gerinnungskaskade kann dabei auf unterschiedliche Weise erfolgen. So kann die Gelierung der Probe festgestellt werden (*gel clot* Test oder Gelierungstest). Andere Verfahren basieren auf Trübungsmessungen oder kinetischen Trübungsmessungen. Andere Verfahren verwenden fluoreszierende Substrate für Enzyme der Gerinnungskaskade.

Das einfachste Verfahren ist der Gelierungstest. Er ist vielseitig anwendbar und ohne großen apparativen Aufwand durchführbar. Außerdem wird er nach der European Pharmacopoeia als der zuverlässigste Nachweis eingestuft. Die Nachweisgrenze liegt für die meisten erhältlichen Tests bei 0,03 EU/ml (Endotoxin Units/ml).

Ein Problem dieses Nachweises ist aber die Interferenz mit vielen unterschiedlichen Substanzen in der Probe. Daher ist es oft notwendig die Proben zu verdünnen, um mögliche Interferenzen zu reduzieren. Allerdings werden damit auch die in der Probe vorhandenen Endotoxine verdünnt. Daher ist die Empfindlichkeit des Nachweises häufig nicht ausreichend.

Um die für eine Probe geforderte Empfindlichkeit nicht zu unterschreiten, darf eine Probe nicht über die maximale zugelassene Verdünnung (MVD) hinaus verdünnt werden. Diese errechnet sich aus der Empfindlichkeit des Nachweises und dem für die zu untersuchende Probe vorgegebenen Grenzwert für Endotoxine. Dies führt dazu, dass für manche Proben keine Messung möglich ist, da sie nicht ausreichend verdünnt werden können, um Interferenzen zu vermeiden.

Außerdem benötigt gerade der Gelierungstest eine große Menge an Reagenz, welches aus lebenden Tieren gewonnen werden muss.

Die Gerinnungskaskade kann auch durch andere Substrate ausgelöst werden. So können durch den beschriebenen Nachweis auch 1,3-β-D-Glucane (1,3-beta-D-Glucane) nachgewiesen werden.

Aufgabe

Aufgabe der Erfindung ist es, ein Verfahren bereitzustellen, das die Empfindlichkeit des Nachweises für Endotoxine und/oder 1,3-β-D-Glucane verbessert.

Lösung

Diese Aufgabe wird durch die Erfindungen mit den Merkmalen der unabhängigen Ansprüche gelöst. Vorteilhafte Weiterbildungen der Erfindungen sind in den Unteransprüchen gekennzeichnet. Der Wortlaut sämtlicher Ansprüche wird hiermit durch Bezugnahme zum Inhalt dieser Beschreibung gemacht. Die Erfindungen umfassen auch alle sinnvollen und insbesondere alle erwähnten Kombinationen von unabhängigen und/oder abhängigen Ansprüchen.

Die Erfindung betrifft ein Verfahren zur Detektion von Endotoxinen und/oder 1,3-β-D-Glucanen in einer Probe umfassend folgende Schritte:

a) Kontaktierung der Probe mit einem Amöbozyten-Lysat und mindestens einer Art von Partikeln, welche eine Oberfläche aus mindestens einem Metall- oder Halbmetalloxid aufweisen, wobei die Partikel keine Oberflächenmodifizierung aufweisen, ein Zetapotential in Wasser von unter -30 mV aufweisen, in der kontaktierten Probe in einer Konzentration von unter 500 μg/ml vorliegen und eine primäre Partikelgröße von unter 300 nm aufweisen;

b) Überprüfen der Probe auf eine Veränderung durch optische Verfahren, wobei das Eintreten einer Veränderung die Anwesenheit von Endotoxinen und/oder 1,3-β-D-Glucanen des Endotoxins nachweist.

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen.

Die Erfindung betrifft die Detektion von Endotoxinen und/oder 1,3-β-D-Glucanen. Im Sinne der Erfindung wird unter einem Endotoxin ein pyrogener Bestandteil der Zellmembran von gramnegativen Bakterien verstanden. Es handelt sich um Lipopolysaccharide (LPS).

Unter einem 1,3-β-D-Glucan wird jedes wasserlösliche Polysaccharid oder Derivat davon verstanden, welches die Gerinnungskaskade in natürlichem LAL auslösen kann und mindestens zwei Glucose-Moleküle, welche β-1,3-glycosidisch verbunden sind, aufweist. Das Polysaccharid kann auch noch weitere Glycoside aufweisen, welche auch auf andere Weise miteinander verknüpft sein können. In einem ersten Schritt wird die Probe mit einem Amöbozyten-Lysat und mindestens einer Art Partikel kontaktiert. Bei dem Amöbozyten-Lysat handelt es sich um ein Lysat, welches vor der Kontaktierung oder während der Kontaktierung rekonstituiert wird. Die Rekonstitution kann erforderlich sein, wenn das Lysat zum Beispiel gefriergetrocknet gelagert wird. Die Kontaktierung besteht dabei vorzugsweise aus der Herstellung einer Mischung aus allen eingesetzten Komponenten.

Überraschenderweise wurde gefunden, dass die Zugabe der Partikel die Empfindlichkeit des Nachweises deutlich steigern kann. Dies bietet nicht nur den Vorteil, dass eine Probe stärker verdünnt werden kann, sondern es reduziert auch den Verbrauch an Reagenz. Gleichzeitig könnte es auch zu einer Beschleunigung des Nachweises kommen. Im Falle des Gelierungstest benötigt der Nachweis üblicherweise 1 Stunde.

Die Reihenfolge der Zugabe der Komponenten ist nicht entscheidend. Es ist möglich zunächst die Nanopartikel in die Probe zu geben und erst danach das Lysat hinzuzufügen. Bevorzugt wird das Lysat als letzter Bestandteil zugegeben. Die Partikel können auch zunächst in das Lysat gegeben werden. Das Verfahren kann auch noch die Zugabe weiterer Komponenten umfassen.

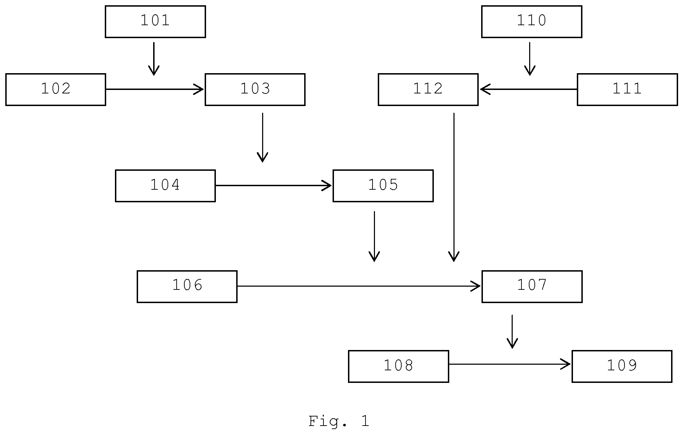

Unter Amöbozyten-Lysat wird im Sinne der Erfindung jedes Lysat oder Teil davon verstanden, welches aus Pfeilschwanzkrebsen gewonnen und/oder danach *in vitro* daraus hergestellt wurde. Das Lysat kann auch nur oder zusätzlich ein oder mehrere isolierte oder rekombinant hergestellte Komponenten der Gerinnungskaskade des Pfeilschwanzkrebses umfassen. Das Amöbozyten-Lysat wurde bevorzugt aus Pfeilschwanzkrebsen gewonnen und/oder besteht aus isolierten oder rekombinanten Bestandteilen der Gerinnungskaskade des Pfeilschwanzkrebses. Dies sind bevorzugt die Enzyme aus Figur 1.

Das Amöbozyten-Lysat (AL) ist bevorzugt ein Lysat, hergestellt aus der Hämolymphe von Pfeilschwanzkrebsen (*Limulidae*), besonders bevorzugt *Limulus polyphemus, Tachypleus gigas, Tachypleus tridentatus* und *Carcinoscorpius rotundicauda*. Bevorzugt ist das Lysat von *Limulus polyphemus* (LAL) und *Tachypleus tridentatus* (TAL).

Figur 1 zeigt eine schematische Darstellung der Gerinnungskaskade bei der Detektion von Endotoxinen oder 1,3-β-D-Glucanen. Ein Endotoxin 101 aktiviert zunächst Faktor C 102 zu 103. Dieser wirkt auf einen Faktor B ein (104 → 105: aktivierter Faktor B). Dieser aktivierte Faktor B 105 aktiviert das *"proclotting enzyme"* 106, das Gelierungsenzym 107 (*"clotting enzyme"*) zu produzieren. Dieses Enzym hydrolysiert spezifische Positionen von Coagulogen 108, um Coagulin 109 zu erzeugen. Dies führt zur Gelierung oder Trübung der Probe. Diese Aktivierung wird auch als "Faktor C Pfad" bezeichnet.

Die Gerinnungskaskade kann auch auf einem anderen Weg durch 1,3-β-D-Glucane ausgelöst werden. Ein solches reaktives Glucan (110) aktiviert dabei einen Faktor G (111 → 112). Dieser aktiviert das *"proclotting enzyme"* 106 und führt so zur Gelierung der Probe. Diese Aktivierung wird auch als "Faktor G Pfad" bezeichnet.

Das Lysat gemäß der Erfindung kann auch nur Teile der Gerinnungskaskade aufweisen. So ist es beispielsweise möglich, einen der beiden Reaktionspfade ausgehend von Endotoxinen oder 1,3-β-D-Glucanen zu unterdrücken oder die entsprechenden Enzyme zu entfernen, z.B. Faktor G, um eine Interferenz mit 1,3-β-D-Glucanen zu vermeiden. Ebenso kann die Aktivität des entsprechenden Reaktionspfads reduziert sein. Dies kann auch durch die Herstellung des Lysats beeinflusst werden.

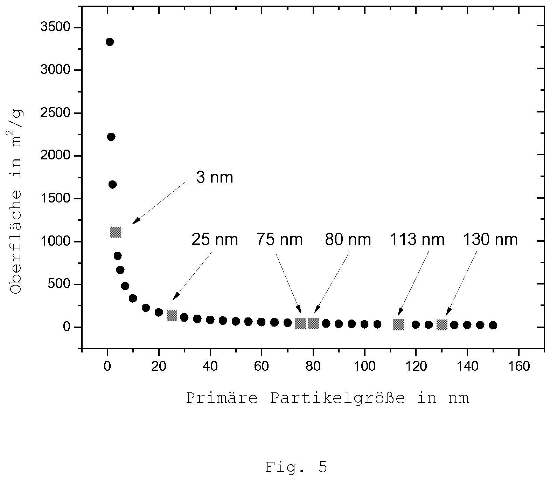

Das Lysat kann auch rekombinant hergestellte Enzyme der Gerinnungskaskade umfassen, z.B. der Enzyme aus Figur 5. So kann beispielsweise rekombinanter Faktor C eingesetzt werden. In diesem Fall kann die zu erkennende Veränderung durch ein spezifisches Substrat des aktivierten Faktor C erreicht werden.

Die Probe wird mit mindestens einer Art von Partikeln mit einer primären Partikelgröße von unter 300 nm kontaktiert.

Unter einer Art von Partikeln werden Partikel verstanden, welche sich in ihrer Zusammensetzung, Größe und Morphologie gleichen.

Die Partikel können beliebige Formen haben. Sie können plättchenförmig, faserförmig, stabförmig oder sphärisch sein. Sie können amorph, porös oder kristallin sein.

Die Partikel weisen eine Oberfläche aus mindestens einem Metall- oder Halbmetall auf. Dies bedeutet, dass mindestens ein Teil ihrer Oberfläche aus mindestens einem Metall- oder Halbmetalloxid besteht. Die Partikel können vollständig aus einem Metall- oder Halbmetalloxid bestehen.

Das mindestens eine Metall- oder Halbmetalloxid ist bevorzugt ausgewählt aus der Gruppe umfassend die Oxide von Mg, Ca, Se, Ba, Al, Si, Sn, Pb, Bi, Ti, Zr, V, Mn, Nb, Ta, Cr, Mo, W, Fe, Co, Fu, Cu, Zn, Ce und Y. Bevorzugt ist das mindestens eine das Metall- oder Halbmetalloxid ausgewählt aus der Gruppe enthaltend Siliziumdioxid, Titandioxid oder Zirkoniumdioxid. Bevorzugt ist es Siliziumdioxid oder Titandioxid, besonders bevorzugt Siliziumdioxid. In einer bevorzugten Ausführungsform handelt es sich um amorphes Siliziumdioxid.

Die Partikel können auch noch andere Materialien, vorzugsweise anorganische Stoffe wie Oxide, umfassen. Es kann sich beispielsweise um Trägermaterialien handeln, welche mindestens teilweise mit dem mindestens einem Metall- oder Halbmetalloxid beschichtet sind. Als Trägermaterialien können unterschiedliche Materialien verwendet werden. Es kann sich um organische oder anorganische Materialien handeln.

Im Falle von organischen Materialien kann es sich um Partikel oder Plättchen aus einem organischen Polymer handeln.

Im Falle von anorganischen Materialien als Trägermaterialien kann es sich um Partikel oder Plättchen aus Oxiden, Sulfiden, Seleniden, Telluriden und/oder Phosphiden handeln. Bevorzugt sind Oxide von Metallen- und Halbmetallen wie z. B. Mg, Ca, Se, Ba, Al, Si, Sn, Pb, Bi, Ti, Zr, V, Mn, Nb, Ta, Cr, Mo, W, Fe, Co, Fu, Cu, Zn, Ce und Y. Es kann sich auch um Partikel handeln, welche mehrere Oxide enthalten. Bevorzugt handelt es sich um Eisenoxid-Partikel. Besonders bevorzugt handelt es sich bei den Trägermaterialien um Eisenoxidpartikel mit einer primären Partikelgröße von unter 10 nm.

In einer bevorzugten Ausführungsform sind die Trägermaterialien vollständig mit dem Metall- oder Halbmetalloxid, bevorzugt mit Siliziumdioxid beschichtet. Es handelt sich um Kern-Schale-Partikel, deren Schale aus dem Metall- oder Halbmetalloxid besteht.

Die Beschichtung der Trägermaterialien und Herstellung der Partikel erfolgt bevorzugt nach dem Sol-Gel-Verfahren. Dieses Verfahren beruht auf der sauren oder alkalischen Hydrolyse von Matrixbildnern. Bei den Matrixbildnern handelt es sich um hydrolysierbare Vorläuferverbindungen des mindestens einen Metall- oder Halbmetalloxids. Dies können zum Beispiel Halogenide oder Alkoxide sein. Als Matrixbildner werden bevorzugt Verbindungen der Formel I

$$SiX_4 \qquad (I)$$

verwendet, wobei X gleich oder verschieden sein können und eine hydrolysierbare Gruppe darstellt, ausgewählt aus der Gruppe der Halogenide (Cl, Br, I) oder Alkoxide ($C_1$-$C_8$-Alkoxide). Beispiele für solche Verbindungen sind Tetramethoxysilan oder Tetraethoxysilan.

Bei Verwendung der Trägermaterialien kommt es dabei zu einer Aufkondensation einer Metall- oder Halbmetalloxidschicht, bevorzugt einer Siliziumdioxidschicht, auf der Oberfläche des Trägermaterials. Die erfindungsgemäßen Partikel weisen auf der Metall- oder Halbmetalloxid-Oberfläche keine Oberflächenmodifizierung auf. Das Lysat muss mit dem Metall- oder Halbmetalloxid interagieren können. Eine Oberflächenmodifikation vermindert die Interaktion zwischen den Proteinen des Nachweises und der Oberfläche aus Metall- oder Halbmetalloxid. Die Oberflächenmodifikation führt daher zu einer geringeren Erhöhung der Empfindlichkeit des Nachweises als durch unmodifizierte Partikel. Dies ist durch Vergleichsversuche einfach zu ermitteln.

In einer Weiterbildung der Erfindung weisen die Partikel eine spezifische Oberfläche von über 10 $m^2$/g, bezogen auf das Metall- oder Halbmetalloxid, auf.

In einer Weiterbildung der Erfindung weisen die Partikel eine primäre Partikelgröße (gemessen mit TEM/SEM) von unter 300 nm, besonders bevorzugt von unter 250 nm. Die primäre Partikelgröße liegt dabei bevorzugt zwischen 2 nm und 200 nm.

In einer Weiterbildung der Erfindung handelt es sich um Nanopartikel. Dies sind Partikel, welche eine primäre Partikelgröße oder Primärteilchengröße (gemessen mit TEM/SEM) von unter 100 nm aufweisen. Bevorzugt sind Partikel mit einer primären Partikelgröße von unter 50 nm, bevorzugt unter 10 nm. Die primäre Partikelgröße der Nanopartikel kann auch zwischen 2 nm und 50 nm liegen.

Die Partikel können auch anhand ihres hydrodynamischen Durchmessers (gemessen in Wasser mit DLS) charakterisiert werden. Der hydrodynamische Durchmesser liegt bevorzugt unter 500 nm, besonders bevorzugt unter 200 nm, inklusive 200 nm. Der hydrodynamische Durchmesser liegt bevorzugt zwischen 10 nm und 200 nm.

Die Partikel sind bevorzugt auf die primäre Teilchengröße redispergierbar. Sie liegen nicht als Aggregate vor.

Es kann nur eine Art von Partikeln verwendet werden. Alternativ können aber auch zwei, drei, vier oder mehr Arten von Partikeln verwendet werden. Bevorzugt erfüllen alle im Nachweis verwendeten Arten von Partikeln die erfindungsgemäßen Anforderungen.

Die Partikel weisen ein Zetapotential in Wasser von unter - 30 mV auf, bevorzugt unter -40 mV auf. Besonders bevorzugt ist ein Zetapotential zwischen -40 mV und -45 mV. Das Zetapotential hat einen besonderen Einfluss auf die Interaktion der Partikel mit dem Lysat.

Die Konzentration der Partikel in der kontaktierten Probe liegt unter 500 mg/ml. Sie kann aber auch deutlich geringer gewählt werden. Die Konzentration der Partikel kann einen Einfluss auf die Empfindlichkeit des Nachweises haben. So erhöhen höhere Konzentrationen die Empfindlichkeit des Nachweises. Zu hohe Konzentrationen können den Nachweis auch negativ beeinflussen. Die zulässigen Konzentrationen können vom Fachmann für die verwendeten Partikel durch einfache Vergleichsversuche festgestellt werden. Bevorzugt liegt die Konzentration der Partikel unter 250 $\mu$g/ml, unter 125 $\mu$g/ml oder unter 62,5 $\mu$g/ml. Im Falle von Partikeln mit einer primären Partikelgröße von unter 10 nm liegt die Konzentration bevorzugt unter 100 $\mu$g/ml.

Der Nachweis wird üblicherweise bei einem pH-Wert zwischen 6 und 8 durchgeführt.

Nach der Kontaktierung der Probe wird die Probe auf eine Veränderung überprüft. Diese Veränderung kann unterschiedliche Eigenschaften der Probe betreffen. So kann sich die Konsistenz der Probe verändern (Gelierung). Auch kann sich die Trübung oder die Farbe der Probe verändern.

Die Veränderung der Probe kann mit beliebigen geeigneten Methoden gemessen werden. Dies können optische Methoden sein wie Inaugenscheinnahme nach Umdrehen der Probe, Trübung, Transmission, Absorption oder Fluoreszenz. Die Messung kann zu einem bestimmten Zeitpunkt oder kontinuierlich erfolgen. So kann auch die Kinetik der Veränderung der Probe bestimmt und ausgewertet werden. Es kann auch die Fluoreszenz von eingesetzten Fluoreszenzsonden gemessen werden, welche die Aktivierung der Gerinnungskaskade anzeigen.

Dies können beispielsweise Substrate für einzelne in Figur 1 gezeigte Enzyme der Gerinnungskaskade sein. Solche Substrate können kurze Peptidketten sein, welche mit detektierbaren Sonden oder Vorstufen davon modifiziert sind. Dies können beispielsweise Nitroaniline sein, welche zu Farbstoffen umgesetzt werden können.

Das Vorliegen einer Veränderung der Probe zeigt dabei die Anwesenheit von Endotoxinen und/oder 1,3-$\beta$-D-Glucanen an. Dabei kann es abhängig von dem verwendeten Lysat nötig sein, durch Kontrollexperimente, z.B. Zugabe von Glucanblockern, Verdünnungsreihen, Positivkontrollen und Negativkontrollen, das Ergebnis zu verifizieren. Damit kann auch zwischen Endotoxinen und 1,3-$\beta$-D-Glucanen unterschieden werden. Endotoxine und/oder 1,3-$\beta$-D-Glucane im Sinne der Erfindung umfassen auch nur eine Art von Endotoxin und/oder eine Art von 1,3-$\beta$-D-Glucan.

In einer bevorzugten Ausführungsform der Erfindung sind die Partikel sphärische Siliziumdioxidpartikel mit einer primären Partikelgröße zwischen 30 nm und 150 nm. Es können auch Mischungen aus mindestens zwei Partikeln mit unterschiedlicher Teilchengröße eingesetzt werden.

In einer weiteren bevorzugten Ausführungsform der Erfindung sind die Partikel sphärische Metalloxid-Siliziumdioxid-Kern-Schale-Partikel, bevorzugt Eisenoxid-Siliziumdioxid-Kern-Schale-Partikel, mit einer primären Partikelgröße zwischen 2 und 100 nm. Bei solchen Partikeln kann eine Erhöhung der Empfindlichkeit auf 0,000875 EU/ml erreicht werden. Dies entspricht Faktor 34 im Vergleich zum Standardnachweis.

Die Partikel der Erfindung erhöhen die Empfindlichkeit des *gel clot* Nachweises für Endotoxine bevorzugt auf unter 0,015 EU/ml, 0,007 EU/ml, 0.0035 EU/ml oder 0.00175 EU/ml.

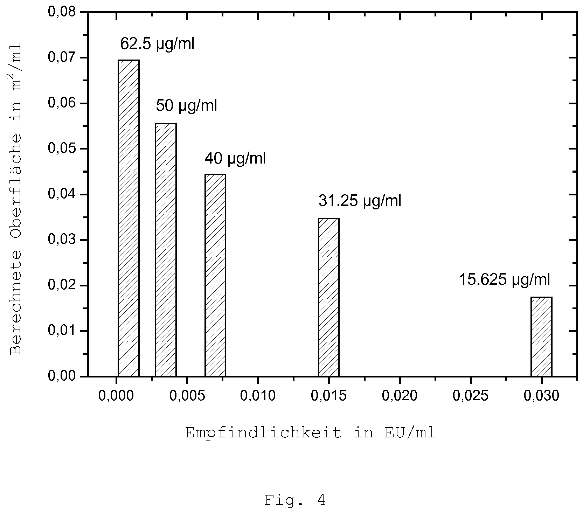

Figur 4 zeigt, dass die Empfindlichkeit in Abhängigkeit von der zur Verfügung stehenden Oberfläche zunimmt. In einer Ausführungsform der Erfindung beträgt die zur Verfügung stehende berechnete Oberfläche der Partikel in dem Nachweis über 0,02 m$^2$/ml, bevorzugt über 0,04 m$^2$/ml.

Das Verfahren kann noch weitere Schritte umfassen. So kann noch die Einstellung des pH-Werts der Probe erforderlich sein.

Außerdem können eine Verdünnung der Probe und Positivkontrollen zur Vermeidung von Interferenzen erforderlich sein. Diese Vorgehensweisen sind dem Fachmann von der üblichen Durchführung des Lysat-Nachweises bekannt.

Das Verfahren kann auch die Zugabe weiterer Additive umfassen. Dies können in Abhängigkeit von der verwendeten Detektionsmethode Flockungsmittel, Detektionssonden (z.B. fluoreszenzmarkierte Substrate für Enzyme des Lysats) oder Puffer sein. Dies kann ein Substrat für den aktivierten Faktor C, "*proclotting enzyme*" oder Gelierungsenzym sein.

Das Verfahren kann auch die Durchführung von Kontrollproben zur Verifizierung der Ergebnisse enthalten. Dies können Positivproben, Negativproben und/oder Verdünnungsreihen sein. Das Verfahren kann auch die Durchführung von IEC umfassen.

Die Erfindung betrifft des Weiteren ein Kit zur Detektion von Endotoxinen. Ein solches Kit umfasst ein Amöbozyten-Lysat und mindestens eine Art von Partikeln, welche eine Oberfläche aus mindestens einem Metall- oder Halbmetalloxid aufweisen, wobei die Partikel keine Oberflächenmodifizierung aufweisen, ein Zetapotential in Wasser von unter -30 mV aufweisen, in der kontaktierten Probe in einer Konzentration von unter 500 $\mu$g/ml vorliegen und eine primäre Partikelgröße von unter 300 nm aufweisen.

Es handelt sich bevorzugt um ein Kit zur Durchführung des erfindungsgemäßen Verfahrens.

Die Partikel können dabei in Suspension oder in trockener Form vorliegen. Das Amöbozyten-Lysat kann rekonstituiert oder in rekonstituierbarer Form vorliegen.

Das Kit kann noch weitere Bestandteile wie Puffer oder Standards enthalten.

Die Erfindung betrifft außerdem die Verwendung von Partikeln, welche eine Oberfläche aus mindestens einem Metall- oder Halbmetalloxid aufweisen, zur Verbesserung der Empfindlichkeit eines Nachweises von Endotoxinen mit einem Amöbozyten-Lysat, wobei die Partikel keine Oberflächenmodifizierung aufweisen, ein Zetapotential in Wasser von unter -30 mV aufweisen, in der kontaktierten Probe in einer Konzentration von unter 500 μg/ml vorliegen und eine primäre Partikelgröße von unter 300 nm aufweisen.

Es handelt sich bevorzugt um die für das Verfahren beschriebenen Partikel.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Materialien und Methoden

Abkürzungen:

EU       Endotoxin Unit
IEC      inhibition/enhancement control
λ         Empfindlichkeit des Assays/Nachweises
MVD    Maximale erlaubte Verdünnung einer Probe, deren Endotoxingehalt noch zuverlässig bestimmt werden kann.
LAL     Limulus-Amöbozyten-Lysat
LPS     Lipopolysaccharide

1. Herstellung der Nanopartikel

Die Siliziumdioxid-Partikel wurden mit einem modifiziertem Stöber-Verfahren (Stoeber et al., 1968) oder durch L-Argininkatalysierte Hydrolyse von Tetraethoxysilan (TEOS) in einem zweiphasigem Wasser/Cyclohexan-System hergestellt (nach Hartlen et al. 2008). Die Eisenoxid-Kerne für die mit Siliziumdioxid-beschichteten Partikel wurden von Nanogate AG (Quierschied-Göttelborn, Germany) bezogen und mit einem modifiziertem Stöber-Verfahren mit Siliziumdioxid beschichtet. Eine der verwendeten Nanopartikel enthielten ein Fluoreszenzlabel (f) mit dem tiefrotem Fluoreszenzfarbstoff Atto647N-NHS. Dazu wurde an den Farbstoff ein Silanlinker gekoppelt. Der so modifizierte Farbstoff wurde bei der Synthese der Partikel in das Wasser/Cyclohexan-System gegeben und auf diese Weise in die Siliziumdioxid-Matrix der Partikel integriert. Die Modifizierung mit PEG wurde durch Aufkondensation eines mit mPEG750 modifiziertem Silanlinker auf der Oberfläche der Partikel erreicht. Alle Chemikalien für die Synthese der Partikel wurden von Sigma-Aldrich (Schnelldorf, Deutschland) bezogen. Atto647N-NHS Ester (NHS: N-Hydroxysuccinimid-Ester) wurde von Atto-Tec (Siegen, Deutschland) bezogen. Endorem®, eine Zusammensetzung von mit Dextran beschichteten, superparamagnetischen EisenoxidNanopartikeln, welche als Kontrastmittel für Magnetresonanztomographie (MRI magnet resonance imaging) verwendet wird, wurde von Guerbet GmbH (Sulzbach, Germany) bezogen und als Referenzmaterial verwendet. Nach der Herstellung wurden alle Partikel mit Hilfe von Dialyse gegen endotoxinfreies Milli-Q-Wasser gereinigt, gefolgt von einer sterilen Filtration auf einer sterilen Arbeitsfläche mit Zellulosemembranen mit 0,2 μm Porengröße. Die Partikel wurden bei 4-8 °C bis zu ihrer Verwendung aufbewahrt. Die Partikel und ihre Eigenschaften sind in Tabelle 1 angegeben.

2. Charakterisierung der Partikel

Zur Charakterisierung der Partikel wurden Aliquoten von Suspensionen der steril filtrierten Partikel eingesetzt. Die Teilchengröße und Morphologie wurden durch Rasterelektronenmikroskopie (REM; SEM scanning electron microscopy) und Transmissionselektronenmikroskopie (TEM) ermittelt. Für SEM wurden unverdünnte Proben der Suspensionen auf Siliziumoberflächen aufgebracht und im Vakuum getrocknet. Der hydrodynamische Durchmesser der Partikel wurde mit dynamischer Lichtstreuung (DLS, dynamic light scattering, Dyna Pro Titan, Wyatt Technology Europe GmbH) bestimmt. Das Zeta-(ζ)-Potential der Partikel wurde in Proben von 1 ml in Einwegküvetten bei 25 °C mit einem Zetasizer Nano (Malvern, Deutschland) gemessen. UV-vis-Spektren in dem Wellenlängenbereich von 350 - 800 nm wurden mit einem Cary 5000 Spektrometer (Varian Inc., Deutschland) aufgenommen. Der pH-Werte aller Proben wurde gemessen und lag in dem für den Assay vorgeschriebenen Bereich (pH 6-8). Eine Einstellung des pH-Werts war daher nicht nötig.

3. Bestimmung der Endotoxine

Endotoxintests wurden mit dem LAL *Gel Clot* Assay Lonza durchgeführt (Pyrogent™ Plus N294-03, Lonza Walkersville Inc., Walkersville, MD, USA). Alle verwendeten Materialien waren steril und gemäß Herstellerangaben pyrogenfrei. Die Tests wurden gemäß dem Protokoll "Nanokon SOP 2.2.2: Detection and semi-Quantification of Endotoxin Contaminations in Nanoparticle Suspensions - Limulus amebocyte lysate (LAL) *Gel Clot* Assay" veröffentlicht auf der the DaNa homepage www.nanopartikel.info (http://nanopartikel.info/files/content/dana/Dokumente/Projekte /SOPs/Nanokon%20SOP%202%202%202 gesch%C3%BCtzt.pdf Kucki, 2012) durchgeführt. Dieses Protokoll basiert auf den Informationen aus European Pharmacopoeia, Monograph 2.6.14. Bacterial Endotoxins (European Pharmacopoeia, 2005), ISO 29701 (ISO 29701, 2010) und (Dobrovolskaia and Neun, 2011).

Die vom Hersteller angegebene Empfindlichkeit des LAL *gel clot* Assays (0,03 EU/ml; Endotoxin unit pro Milliliter) wurde gemäß den Herstellerangaben kontrolliert und für alle eingesetzten Chargen bestätigt.

Für Suspensionen von Nanopartikeln wurde als Endotoxin Grenzwert ein Wert von 0,5 EU/ml angenommen. Aus einer Empfindlichkeit des Nachweises ($\lambda$) von 0,03 EU/ml und einer Probenkonzentration von 1 mg/ml ergibt sich eine maximal zulässige Verdünnung (MVD) von 16 (MVD = Probenkonzentration * Endotoxin Grenzwert / Empfindlichkeit des Nachweises ($\lambda$)).

Verdünnungen und Kontrollen wurde mit endotoxinfreiem Wasser (LAL reagent water W50, Lonza Walkersville Inc., USA) durchgeführt. Falls in den Proben Endotoxine nachgewiesen wurden, wurden die Proben noch auf eine mögliche Störung des Nachweises durch (1,3)-$\beta$-D-Glucan untersucht. Dazu wurde $\beta$-Glucan-Blocker zugegeben (Beta-G-Blocker Kit N190, Lonza Walkersville Inc., USA).

Der Einfluss der Nanopartikel auf die Sensitivität des Assays wurde außerdem durch IEC (inhibition/enhancement controls) überprüft. Diese wurde nach der vorstehend genannten SOP und den Herstellerangaben durchgeführt. Als endotoxinfrei getestete Partikelsuspensionen wurden mit einem Referenzstandard versetzt (certified reference standard endotoxin CSE Escherichia coli 055:B5 Lonza Walkersville Inc., USA) und die Proben untersucht.

Alle Messungen wurden mit unabhängigen Verdünnungsreihen wiederholt.

4. Beispielhafte Durchführung des Verfahrens

4.1 Rekonstitution des LAL

Lyophilisiertes LAL wurde in endotoxinfreiem Wasser rekonstituiert und für mindestens 30 Sekunden aufgewirbelt (nicht geschüttelt). Das rekonstituierte LAL wurde möglichst bald verwendet.

4.2 Nachweis der Endotoxine (Testprozedur)

Alle Proben, Standards oder Kontrollen wurden 1:1 (Vol:Vol) mit rekonstituiertem LAL gemischt (je 100 $\mu$l) und für 1 Stunde bei 37 °C in einem nicht zirkulierendem Wasserbad erwärmt. Die Proben wurden dabei nicht bewegt. Danach wurden die Proben vorsichtig um 180° umgedreht. Eine feste geronnene Masse nach dem Umdrehen zeigt ein positives Testergebnis an. Bei einer negativen Reaktion ist keine geronnene Masse vorhanden, sondern nur eine Flüssigkeit.

4.3 Kontrolle der Empfindlichkeit des Nachweises

Zunächst wurde eine Lösung mit 1 EU/ml Endotoxin verwendet, um eine zweifache Verdünnungsreihe gemäß der vom Hersteller angegebenen LAL-Empfindlichkeit ($\lambda$) herzustellen ($2\lambda$, $1\lambda$, $0,5\lambda$, $0,25\lambda$). Zusätzlich wurde eine Negativkontrolle mit Wasser (LAL reagent water) angefertigt.

Die Proben wurden entsprechend der Testprozedur untersucht. Aus den Ergebnissen wurde das geometrische Mittel der Empfind-lichkeit gebildet. ( $\log x_{geom} = \frac{1}{n} \sum_{i=1}^{n} x_i$ mit $x_i$: Empfindlichkeit; n: Anzahl der Replikate des Nachweises). Das errechnete geometrische Mittel sollte der vom Hersteller angegebenen Empfindlichkeit entsprechen.

4.4 Untersuchung der Nanopartikelsuspensionen

Aus der zu testenden Suspension (1 mg/ml) wurde eine zweifache Verdünnungsreihe (1/2, 1/4, 1/8, 1/16) hergestellt. Aus der 1 EU/ml Endotoxinlösung wurde eine Positivkontrolle mit $2\lambda$ (0,06 EU/ml) hergestellt. Als Negativkontrolle wurde endotoxinfreies Wasser (LAL reagent water) verwendet. Die Proben wurden entsprechend der Testprozedur untersucht.

Aus der positiven Probe mit dem höchsten Verdünnungsfaktor wurde die Konzentration der Endotoxine in der Probe berechnet (Konzentration = Probe * $\lambda$).

Wenn keine Probe positiv war, wurde angenommen, dass die Endotoxin-Konzentration unter der Empfindlichkeit des Assays lag.

4.5 Untersuchung des Einflusses der Nanopartikel auf den Assay (IEC)

Um den Einfluss der Nanopartikel auf den Assay zu untersuchen, wurde die Empfindlichkeit des Assays gemäß der vorstehenden Prozedur für unterschiedliche Konzentrationen der Nanopartikel untersucht. Dafür wurde aus einer 1 EU/ml Endotoxinlösung eine zweifache Verdünnungsreihe hergestellt (2λ, 1λ, 0,5λ, 0,25λ). Dabei wurde zur Verdünnung kein endotoxinfreies Wasser sondern die Suspension der Nanopartikel verwendet. Danach wurde die Empfindlichkeit wie unter Punkt 4.3 beschrieben untersucht.

IEC ist auch bei dem üblichen Verfahren vorgeschrieben, um Fehlmessungen aufgrund von Inhibition oder Verstärkungseffekten zu minimieren. Gemäß der SOP wird der Test als unbeeinflusst gewertet, wenn die gemessene Empfindlichkeit im Bereich von 0,5λ und 2λ liegt (IEC Kriterien). IEC wurden bei MVD und mindestens einer weiteren Partikelkonzentration durchgeführt.

4.6 1,3-β-D-Glucan Interferenztest

Wenn Endotoxine festgestellt wurden, wurde ein 1,3-β-D-Glucan Interferenztest durchgeführt, um eine Interferenz mit 1,3-β-D-Glucan auszuschließen.

Dazu wurde eine Probe der Nanopartikel mit doppelter Testkonzentration hergestellt. Dazu wurde im Verhältnis 1:1 β-G-Blocker (Lonza) zugegeben. Die Probe wurde wie vorstehend angegeben untersucht. Als Kontrollen wurden Proben der Nanopartikel ohne β-G-Blocker, β-G-Blocker in endotoxinfreiem Wasser, Positivprobe mit Endotoxin (2λ) und β-G-Blocker verwendet.

5. Experimente

Fig. 2    REM Abbildung von monodispersen Siliziumdioxidpartikeln (Silica-4-130);

Fig. 3    TEM Abbildung von Silica-2; Mischung von Partikeln mit zwei Größen: kleine Nanopartikel mit 42 nm primärer Partikelgröße (72%) und große Nanopartikel mit einem Durchmesser von ca. 108 nm (28%);

Fig. 4    Empfindlichkeit des LAL *gel clot* Assays in Abhängigkeit von der verwendeten Konzentration der EisenoxidSiliziumdioxid-Partikel $Fe_xO_y@SiO_2$-1 und der berechneten Oberfläche in $m^2$/ml. Die vom Hersteller angegebene Empfindlichkeit des Assays liegt bei 0,03 EU/ml.

Fig. 5    Berechnete Oberfläche der Partikel als Funktion der primären Partikelgröße.

Tabelle 1 zeigt die untersuchten Nanopartikelsuspensionen. In Tabelle 2 sind die Ergebnisse der Untersuchung dieser Suspensionen auf Nanopartikel angegeben. Zwar wurden bei einigen Proben Endotoxine mit einem Gehalt von > 0,03 EU/ml festgestellt, aber die IEC bei MVD zeigten bei fast allen Proben eine Verstärkung der Empfindlichkeit des Nachweises. Bei den positiven Proben konnte nachgewiesen werden, dass keine β-Glucan-Interferenz vorlag. Eine Verunreinigung der Proben mit Endotoxinen konnte aufgrund der Herstellung der Nanopartikel und der Kontrollexperimente ausgeschlossen werden.

Die in Tabelle 1 in eckigen Klammern angegebenen Werte sind im Vergleich zu einem Nachweis ohne Nanopartikel keine zulässigen Messwerte, da für sie die IEC nicht die angegebenen Akzeptanzkriterien erfüllte. Dies zeigt, dass die Empfindlichkeit des Nachweises durch die Zugabe dieser Nanopartikel besonders stark erhöht wurde.

5.1 Siliziumdioxid-Partikel

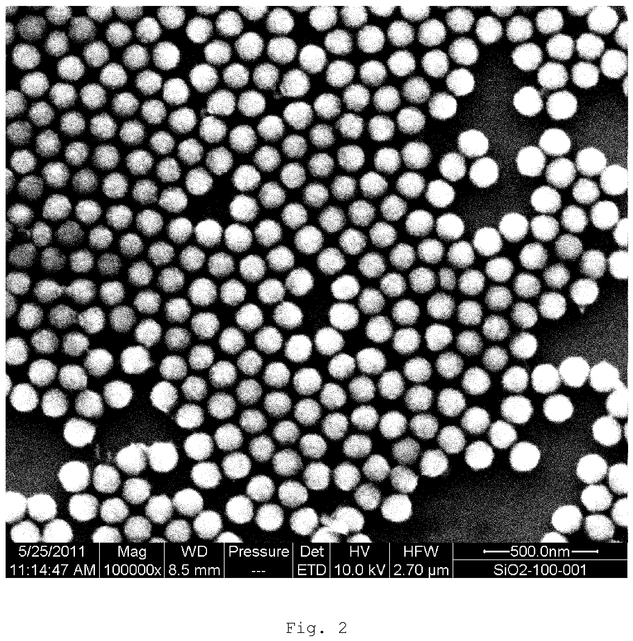

Wenn Proben mit einer Konzentration von 62,5 μg/ml (dies entspricht MVD für Nanopartikel) gemessen wurde, zeigten nur Endorem und Silica-1-25 keine Veränderung der Empfindlichkeit des Nachweises. Allerdings hatten die Silica-1-25-Partikel auch ein deutlich verändertes Zeta-Potential von -24,2 mV. Vier der getesteten Siliziumdioxid-Partikel (Silica-2, Silica-3-80, Silica-5[f], Silica-6+PEG[f]) zeigten eine Verstärkung der Empfindlichkeit, aber innerhalb der Grenzen der IEC. Nur Silica-4-130 (siehe auch Fig. 2), eine Suspension mit monodispersen Siliziumdioxid-Teilchen mit einer primären Teilchengröße von 130 nm, zeigte eine höhere Verstärkung als gemäß IEC zulässig, wenn es innerhalb des zulässigen Verdünnungsbereichs (bis zu MVD) untersucht wurde.

Mit Silica-3-80 wurden konzentrationsabhängige IEC durchgeführt. Diese ergaben eine Verbesserung der Empfindlichkeit des Nachweises auf 0,015 EU/ml für alle untersuchten Partikelkonzentrationen (500 μg/ml, 250 μg/ml, 125 μm/ml, 62,5 μg/ml). Allerdings wurde keine Veränderung der Empfindlichkeit in Bezug auf die eingesetzte Menge der Partikel festgestellt.

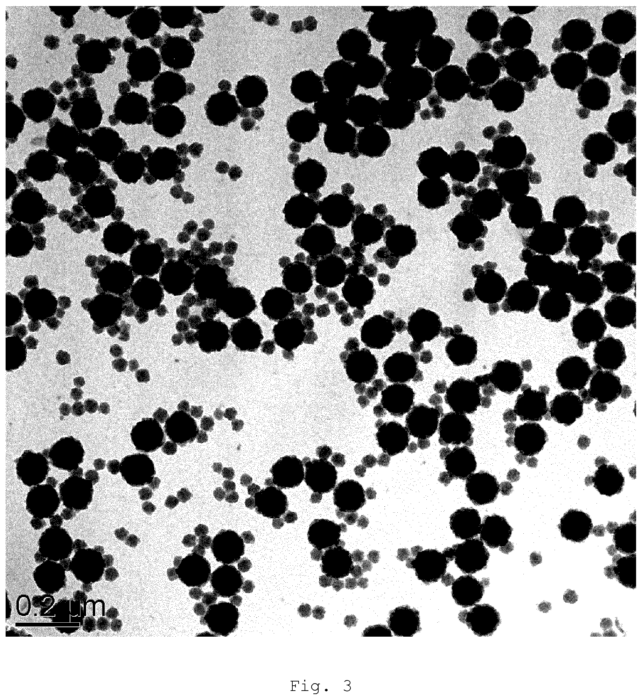

Tabelle 3 zeigt konzentrationsabhängige IEC für Silica-2 (Fig. 3). Dabei steht + für einen positiven Nachweis und - für einen negativen Nachweis. Eine Verringerung der Konzentration der Partikel führte in diesem Fall auch zu einer Verschlechterung der Empfindlichkeit des Nachweises.

Um den Einfluss von Oberflächenmodifikation zu untersuchen, wurden IEC mit Silica-5[f] und Silica-6+PEG[f] durchgeführt. Diese Partikel haben eine ähnliche Größe. Es zeigte sich, dass bei einer hohen Partikelkonzentration (500 μg/ml SiO$_2$) die Verstärkung der Empfindlichkeit unterschiedlich war. Mit PEG modifizierte Partikel Silica-6+PEG[f] zeigten eine geringere Verstärkung innerhalb der IEC Akzeptanzkriterien. Die Verstärkung im Falle der Silica-5[f] lag dagegen außerhalb der Akzeptanzkriterien. Dies deutet darauf hin, dass die Verstärkung durch Oberflächenmodifizierung der Partikel mit organischen Gruppen reduziert wird.

5.2 Eisenoxid-Siliziumdioxid-Kern-Schale-Partikel

Die Eisenoxid-Siliziumdioxid-Kern-Schale-Partikel Fe$_x$O$_y$@SiO$_2$-1, Fe$_x$O$_y$@SiO$_2$-2[f] und Fe$_x$O$_y$@SiO$_2$-3[f] wurden mit dem gleichen Verfahren hergestellt. Im Falle der fluoreszenzmarkierten Partikel wurde lediglich ein Fluoreszenzfarbstoff wie bereits beschrieben in die Partikel eingebaut. Alle Partikelsuspensionen zeigten ähnliches Verhalten im LAL *gel clot* Assay. Es wurden keine Unterschiede aufgrund der Fluoreszenzmarkierung festgestellt.

Bei hohen Konzentrationen (1 mg/ml und 500 μg/ml) wurde eine vollständige Inhibierung des Nachweises festgestellt (keine Gerinnung). Außerdem konnte ein Ausfallen der Partikel beobachtet werden. Bei mittleren Konzentrationen (250 μg/ml und 125 μg/ml) wurde dies nicht beobachtet. Stattdessen formte sich eine bräunliche geronnene Masse. Bei MVD (62,5 μg/ml) war der Test wie erwartet negativ. Der berechnete Gehalt an Endotoxin mit 0,25 EU/ml lag unter dem Endotoxin-Limit von 0,5 EU/ml. Aber die IEC für alle Partikel zeigte eine deutliche Verstärkung der Empfindlichkeit jenseits der IEC Kriterien.

Tabelle 4 zeigt konzentrationsabhängige IEC für Fe$_x$O$_y$@SiO$_2$-1. Die Ergebnisse zeigen eine deutliche Verstärkung der Empfindlichkeit in Abhängigkeit von der Konzentration der Partikel. Bei einer Konzentration von 62,5 μg/ml konnten noch 0,000875 EU/ml nachgewiesen werden. Dies entspricht einer Steigerung um den Faktor 34.

Auch die größeren Partikel Fe$_x$O$_y$@SiO$_2$-4[f] wurden untersucht. Auch sie zeigten eine deutliche Verstärkung der Empfindlichkeit des Nachweises bis zur MVD. Allerdings konnte keine vollständige Inhibierung des Assays bei 500 μg/ml beobachtet werden.

5.3 Endorem ®

Endorem ® ist eine Zusammensetzung aus superparamagnetischen Eisenoxidnanopartikeln (SPION) mit einer primären Partikelgröße von 5 nm. Es ist als Kontrastmittel zur Untersuchung von Lebermetastasen zugelassen. Die Partikel sind mit Dextran oberflächenmodifiziert.

Bei den Untersuchungen konnten keine Endotoxine nachgewiesen werden. Außerdem zeigte Endorem ® keinerlei Wechselwirkung mit dem LAL *gel clot* Assay bei der geringsten verwendeten Konzentration (62,5 μg/ml). Selbst wenn es unverdünnt verwendet wurde (11,2 mg/ml), wurde keine vollständige Inhibierung des Nachweises beobachtet. Auch ein Ausfallen der Partikel wurde nicht beobachtet.

Es sind zahlreiche Abwandlungen und Weiterbildungen der beschriebenen Ausführungsbeispiele verwirklichbar.

Tabelle 1

| Physikalisch-chemische Eigenschaften der untersuchten Nanopartikelsuspeasionen | | | | |
|---|---|---|---|---|
| **Partikel** | **Synthese** | **Hydrodynamischer Durchmesser in nm (DLS)** | **Primäre Partikelgröße in nm** | **Zeta-potential (mV), 25°C, in Wasser** |
| Endorem® | - | 246 | 5 | -35 |
| Fe$_x$O$_y$@SiO$_2$-1 | Stöber | 16,6 ± 5,0 | 3 | -44,7 |
| Fe$_x$O$_y$@SiO$_2$-2[f] | Stöber | 22,1 ± 6,6 | 3 | -42,9 |
| Fe$_x$O$_y$@SiO$_2$-3[f] | Stöber | 88,9 ± 49 | 3 | -43,2 |
| Fe$_x$O$_y$@SiO$_2$-4[f] | Stöber | 71,1 ± 10 | 75 | -41,4 |
| Silica-1-25 | Hartlen | 20,8 ± 2,6 | 24,6 ± 3,4 | -24,2 |
| Silica-2 | Hartlen | 97,6 ± 39 | 42 and 108 | -42,9 |
| Silica-3-80 | Hartlen | 84,2 ± 6,7 | 80 ± 5 | -42,3 |
| Silica-4-130 | Hartlen | 141,4 ± 15 | 128,3 ± 11,0 | -40,5 |

(fortgesetzt)

| Physikalisch-chemische Eigenschaften der untersuchten Nanopartikelsuspeasionen | | | | |
|---|---|---|---|---|
| Partikel | Synthese | Hydrodynamischer Durchmesser in nm (DLS) | Primäre Partikelgröße in nm | Zeta-potential (mV), 25°C, in Wasser |
| Silica-5[f] | Stöber | 104,0 $\pm$ 35 | 113 $\pm$ 14 | -43,2 |
| Silica-6+PEG[f] | Stöber | 96,0 $\pm$ 34 | 113 $\pm$ 14 | -41,2 |
| f     mit Fluoreszenzfarbstoff | | | | |

Tabelle 2

| Ergebnisse der Endotoxintests | | | |
|---|---|---|---|
| Partikel | Endotoxin in EU/ml bei 1 mg/ml Partikel | IEC bei MVD | IEC Kriterien erfüllt? |
| Endorem® | < 0,03 | Angegebene Empfindlichkeit | + |
| $Fe_xO_y@SiO_2$-1 | [0,25] | Verstärkung | - |
| $Fe_xO_y@SiO_2$-2[f] | [0,25] | Verstärkung | - |
| $Fe_xO_y@SiO_2$-3[f] | [0,25] | Verstärkung | - |
| $Fe_xO_y@SiO_2$-4[f] | [0,25] | Verstärkung | - |
| Silica-1-25 | < 0,03 | Angegebene Empfindlichkeit | + |
| Silica-2 | < 0,03 | Verstärkung | + |
| Silica-3-80 | < 0,03 | Verstärkung | + |
| Silica-4-130 | [0,06] | Verstärkung | - |
| Silica-5[f] | < 0,03 | Verstärkung | + |
| Silica-6+PEG[f] | < 0,03 | Verstärkung | + |
| [ ] = Messwerte nicht akzeptabel, da IEC Kriterien nicht erfüllt. | | | |

Tabelle 3

| Konzentrationsabhängige IEC für Silica-2 | | | | | |
|---|---|---|---|---|---|
| Partikelkonzentration in µg/ml | IEC Endotoxin Wert in EU/ml | | | | |
| | 0,03 | 0,015 | 0,007 | 0,0035 | NegativKontrolle |
| 1000 | + | + | + | + | - |
| 500 | + | + | + | + | - |
| 250 | + | + | + | - | - |
| 125 | + | + | - | - | - |
| 62,5 | + | + | - | - | - |

Tabelle 4

| Konzentrationsabhängige IEC für Fe$_x$O$_y$@SiO$_2$-1 | | | | | | |
|---|---|---|---|---|---|---|
| Partikelkonzentration in µg/ml | IEC Endotoxin Wert in EU/ml | | | | | |
| | 0,03 | 0,015 | 0,007 | 0,0035 | 0,00175 | 0,000875 |
| 62,5 | + | + | + | + | + | + |
| 50 | + | + | + | + | - | ND |
| 40 | + | + | + | - | - | ND |
| 31,25 | + | + | - | - | - | - |
| 15,625 | + | - | - | ND | ND | ND |
| ND = nicht gemessen | | | | | | |

zitierte Literatur

STOEBER, W., FINK, A. & BOHN, E. 1968. Controlled Growth of Monodisperse Silica Spheres in the Micron Size Range. Journal of Colloid and Interface Science, 26, 62-69.

HARTLEN, K. D., ATHANASOPOULOS, A. P. T. & KITAEV, V. 2008. Facile Preparation of Highly Monodisperse Small Silica Spheres (15 to >200 nm) Suitable for Colloidal Templating and Formation of Ordered Arrays. Langmuir, 24, 1714-1720.

Bezugszeichen

101 Endotoxin
102 Faktor C
103 aktivierter Faktor C
104 Faktor B
105 aktivierter Faktor B
106 *"proclotting enzyme"*
107 Gelierungsenzym
108 Coagulogen
109 Coagulin
110 1,3-b-D-Glucan oder anderes LAL aktivierendes Glucan
111 Faktor G
112 aktivierter Faktor G

**Patentansprüche**

1. Verfahren zur Detektion von Endotoxinen und/oder 1,3-β-D-Glucanen in einer Probe umfassend folgende Schritte:

a) Kontaktierung der Probe mit einem Amöbozyten-Lysat und mindestens einer Art von Partikeln, welche eine Oberfläche aus mindestens einem Metall- oder Halbmetalloxid aufweisen, wobei die Partikel keine Oberflächenmodifizierung aufweisen, ein Zetapotential in Wasser von unter -30 mV aufweisen, in der kontaktierten Probe in einer Konzentration von unter 500 µg/ml vorliegen und eine primäre Partikelgröße von unter 300 nm aufweisen;
b) Überprüfen der Probe auf eine Veränderung durch optische Verfahren, wobei das Eintreten einer Veränderung die Anwesenheit von Endotoxinen und/oder 1,3-β-D-Glucanen anzeigt.

**2.** Verfahren nach Anspruch 1, **dadurch gekennzeichnet, dass** die Partikel eine primäre Partikelgröße von unter 250 nm haben.

**3.** Verfahren nach einem der Ansprüche 1 oder 2, **dadurch gekennzeichnet, dass** die Partikel in der kontaktierten Probe in einer Konzentration von unter 250 µg/ml vorliegen.

**4.** Verfahren nach einem der Ansprüche 1 bis 3, **dadurch gekennzeichnet, dass** das Metall- oder Halbmetalloxid ausgewählt ist aus der Gruppe enthaltend Siliziumdioxid, Titandioxid oder Zirkoniumdioxid.

**5.** Verfahren nach Anspruch 4, **dadurch gekennzeichnet, dass** das Metall- oder Halbmetalloxid Siliziumdioxid ist.

**6.** Verfahren nach einem der Ansprüche 1 bis 5, **dadurch gekennzeichnet, dass** die zur Verfügung stehende berechnete Oberfläche der Partikel in dem Nachweis über 0,02 m$^2$/ml beträgt.

**7.** Verfahren nach einem der Ansprüche 1 bis 6, **dadurch gekennzeichnet, dass** das Amöbozyten-Lysat aus Pfeilschwanzkrebsen gewonnen wurde und/oder aus isolierten oder rekombinanten Bestandteilen der Gerinnungskaskade des Pfeilschwanzkrebses besteht.

**8.** Kit zur Detektion von Endotoxinen und/oder 1,3-β-D-Glucanen, **dadurch gekennzeichnet, dass** es ein Amöbozyten-Lysat und mindestens eine Art von Partikeln, welche eine Oberfläche aus mindestens einem Metall- oder Halbmetalloxid aufweisen, umfasst, wobei die Partikel keine Oberflächenmodifizierung aufweisen, ein Zetapotential in Wasser von unter -30 mV aufweisen, in der kontaktierten Probe in einer Konzentration von unter 500 µg/ml vorliegen und eine primäre Partikelgröße von unter 300 nm aufweisen.

**9.** Kit nach Anspruch 8, **dadurch gekennzeichnet, dass** die mindestens eine Art von Partikeln Partikel nach einem der Ansprüche 2 bis 6 sind.

**10.** Verwendung von Partikeln, welche eine Oberfläche aus mindestens einem Metall- oder Halbmetalloxid aufweisen, zur Verbesserung der Empfindlichkeit eines Nachweises von Endotoxinen und/oder 1,3-β-D-Glucanen mit einem Amöbozyten-Lysat, wobei die Partikel keine Oberflächenmodifizierung aufweisen, ein Zetapotential in Wasser von unter -30 mV aufweisen, in der kontaktierten Probe in einer Konzentration von unter 500 µg/ml vorliegen und eine primäre Partikelgröße von unter 300 nm aufweisen.

**11.** Verwendung nach Anspruch 10, **dadurch gekennzeichnet, dass** es Partikel nach einem der Ansprüche 2 bis 6 sind.

**Claims**

**1.** Method for detecting endotoxins and/or 1,3-β-D-glucans in a sample, comprising the following steps:

a) contacting the sample with an amebocyte lysate and at least one type of particles having a surface composed of at least one metal or metalloid oxide, wherein the particles have no surface modification, have a zeta potential in water of below -30 mV, are present in the contacted sample in a concentration of below 500 µg/ml and have a primary particle size of below 300 nm;
b) examining the sample for a change by optical methods, wherein the occurrence of a change indicates the presence of endotoxins and/or 1,3-β-D-glucans.

**2.** Method according to Claim 1, **characterized in that** the particles have a primary particle size of below 250 nm.

**3.** Method according to either of Claims 1 and 2, **characterized in that** the particles are present in the contacted sample in a concentration of below 250 µg/ml.

**4.** Method according to any of Claims 1 to 3, **characterized in that** the metal or metalloid oxide is selected from the group containing silicon dioxide, titanium dioxide or zirconium dioxide.

**5.** Method according to Claim 4, **characterized in that** the metal or metalloid oxide is silicon dioxide.

**6.** Method according to any of Claims 1 to 5, **characterized in that** the available calculated surface area of the particles in the detection is above 0.02 m$^2$/ml.

7.  Method according to any of Claims 1 to 6, **characterized in that** the amebocyte lysate has been obtained from horseshoe crabs and/or consists of isolated or recombinant constituents of the coagulation cascade of the horseshoe crab.

8.  Kit for detecting endotoxins and/or 1,3-β-D-glucans, **characterized in that** it comprises an amebocyte lysate and at least one type of particles having a surface composed of at least one metal or metalloid oxide, wherein the particles have no surface modifications, have a zeta potential in water of below -30 mV, are present in the contacted sample in a concentration of below 500 μg/ml and have a primary particle size of below 300 nm.

9.  Kit according to Claim 8, **characterized in that** the at least one type of particles are particles according to any of Claims 2 to 6.

10. Use of particles having a surface composed of at least one metal or metalloid oxide for improving the sensitivity of a detection of endotoxins and/or 1,3-β-D-glucans with an amebocyte lysate, wherein the particles have no surface modification, have a zeta potential in water of below -30 mV, are present in the contacted sample in a concentration of below 500 μg/ml and have a primary particle size of below 300 nm.

11. Use according to Claim 10, **characterized in that** they are particles according to any of Claims 2 to 6.

**Revendications**

1.  Procédé de détection d'endotoxines et/ou de 1,3-β-D-glucanes dans un échantillon, comprenant les étapes suivantes :

    a) la mise en contact de l'échantillon avec un lysat d'amébocytes et au moins un type de particules, qui comprennent une surface en au moins un oxyde de métal ou de semi-métal, les particules ne comprenant pas de modification de surface, présentant un potentiel zêta dans l'eau de moins de -30 mV, étant présentes dans l'échantillon mis en contact en une concentration de moins de 500 μg/ml et présentant une taille de particule primaire de moins de 300 nm ;
    b) le contrôle de l'échantillon pour détecter une modification par des procédés optiques, l'occurrence d'une modification indiquant la présence d'endotoxines et/ou de 1,3-β-D-glucanes.

2.  Procédé selon la revendication 1, **caractérisé en ce que** les particules ont une taille de particule primaire de moins de 250 nm.

3.  Procédé selon l'une quelconque des revendications 1 ou 2, **caractérisé en ce que** les particules sont présentes dans l'échantillon mis en contact en une concentration de moins de 250 μg/ml.

4.  Procédé selon l'une quelconque des revendications 1 à 3, **caractérisé en ce que** l'oxyde de métal ou de semi-métal est choisi dans le groupe contenant le dioxyde de silicium, le dioxyde de titane ou le dioxyde de zirconium.

5.  Procédé selon la revendication 4, **caractérisé en ce que** l'oxyde de métal ou de semi-métal est le dioxyde de silicium.

6.  Procédé selon l'une quelconque des revendications 1 à 5, **caractérisé en ce que** la surface calculée disponible des particules dans la détection est de plus de 0,02 m$^2$/ml.

7.  Procédé selon l'une quelconque des revendications 1 à 6, **caractérisé en ce que** le lysat d'amébocytes a été obtenu à partir de limules et/ou est constitué de constituants isolés ou recombinants de la cascade de coagulation de la limule.

8.  Kit pour la détection d'endotoxines et/ou de 1,3-β-D-glucanes, **caractérisé en ce qu'**il comprend un lysat d'amébocytes et au moins un type de particules, qui comprennent une surface en au moins un oxyde de métal ou de semi-métal, les particules ne comprenant pas de modification de surface, présentant un potentiel zêta dans l'eau de moins de -30 mV, étant présentes dans l'échantillon mis en contact en une concentration de moins de 500 μg/ml et présentant une taille de particule primaire de moins de 300 nm.

**9.** Kit selon la revendication 8, **caractérisé en ce que** ledit au moins un type de particules correspond à des particules selon l'une quelconque des revendications 2 à 6.

**10.** Utilisation de particules qui comprennent une surface en au moins un oxyde de métal ou de semi-métal pour l'amélioration de la sensibilité d'une détection d'endotoxines et/ou de 1,3-$\beta$-D-glucanes avec un lysat d'amébocytes, les particules ne comprenant pas de modification de surface, présentant un potentiel zêta dans l'eau de moins de -30 mV, étant présentes dans l'échantillon mis en contact en une concentration de moins de 500 $\mu$g/ml et présentant une taille de particule primaire de moins de 300 nm.

**11.** Utilisation selon la revendication 10, **caractérisée en ce qu'**il s'agit de particules selon l'une quelconque des revendications 2 à 6.

```
┌─────────┐                              ┌─────────┐
│   101   │                              │   110   │
└────┬────┘                              └────┬────┘
     │                                        │
     ▼                                        ▼
┌─────────┐    ┌─────────┐        ┌─────────┐    ┌─────────┐
│   102   │───►│   103   │        │   112   │◄───│   111   │
└─────────┘    └────┬────┘        └────┬────┘    └─────────┘
                    │                  │
                    ▼                  │
     ┌─────────┐    ┌─────────┐        │
     │   104   │───►│   105   │        │
     └─────────┘    └────┬────┘        │
                         │             │
                         ▼             ▼
     ┌─────────┐              ┌─────────┐
     │   106   │─────────────►│   107   │
     └─────────┘              └────┬────┘
                                   │
                                   ▼
                   ┌─────────┐    ┌─────────┐
                   │   108   │───►│   109   │
                   └─────────┘    └─────────┘
```

Fig. 1

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **STOEBER, W. ; FINK, A. ; BOHN, E.** Controlled Growth of Monodisperse Silica Spheres in the Micron Size Range. *Journal of Colloid and Interface Science,* 1968, vol. 26, 62-69

- **HARTLEN, K. D. ; ATHANASOPOULOS, A. P. T. ; KITAEV, V.** Facile Preparation of Highly Monodisperse Small Silica Spheres (15 to >200 nm) Suitable for Colloidal Templating and Formation of Ordered Arrays. *Langmuir,* 2008, vol. 24, 1714-1720